# EUROPÄISCHE PATENTSCHRIFT

(11) **EP 2 861 527 B1**
(45) Veröffentlichungstag und Bekanntmachung des Hinweises auf die Patenterteilung: **01.06.2016**
(21) Anmeldenummer: 13728203.4
(22) Anmeldetag: 13.06.2013
(51) Int. Cl.: C01B 3/36, C07C 2/78

(54) **VERFAHREN ZUR HERSTELLUNG VON ACETYLEN UND SYNTHESEGAS**
METHOD FOR PRODUCING ACETYLENE AND SYNTHESIS GAS
PROCÉDÉ DE PRODUCTION D'ACÉTYLÈNE ET DE GAZ DE SYNTHÈSE

(30) Priorität: 14.06.2012 EP 12171956
(43) Veröffentlichungstag der Anmeldung: 22.04.2015
(73) Patentinhaber: BASF SE, 67056 Ludwigshafen am Rhein (DE)
(72) Erfinder: VICARI, Maximilian, 67117 Limburgerhof (DE); WEICHERT, Christian, 67098 Bad Dürkheim (DE); GROßSCHMIDT, Dirk, 68259 Mannheim (DE); RUSS, Michael, 67354 Römerberg (DE); HAIDER, Mirko, 67133 Maxdorf (DE); NEUHAUSER, Horst, 67373 Dudenhofen (DE); HAYES, Michael, L., Gonzales, Louisiana 70737 (US)
(74) Vertreter: BASF IP Association
(86) Internationale Anmeldenummer: PCT/EP2013/062218
(87) Internationale Veröffentlichungsnummer: WO 2013/186291

(56) Entgegenhaltungen:
- WO-A1-2012/010508
- US-A- 5 824 834

## Beschreibung

Die vorliegende Erfindung betrifft ein Verfahren zur Herstellung von Acetylen und Synthesegas durch partielle Oxidation von Kohlenwasserstoffen mit Sauerstoff.

Die obige partielle Oxidation ist eine Hochtemperaturreaktion, die üblicherweise in einem Reaktorsystem, umfassend eine Mischeinrichtung, einen Brennerblock sowie eine Quencheinrichtung, durchgeführt wird, und beispielsweise in Ullmanns Encyclopedia of Industrial Chemistry (5th Edition, Volume A1, Seiten 97-144) oder US 005824834A beschrieben ist.

Gemäß Ullmanns Encyclopedia of Industrial Chemistry (5th Edition, Volume A1, Seiten 97-144) erfolgt die Aufheizung der Einsatzstoffe getrennt in Vorheizern. Die aufgeheizten Einsatzstoffe werden in einer Mischeinrichtung gemischt und über einen Mischdiffusor einem Brenner und weiter einem Feuerraum zugeführt. Stromab des Feuerraums wird mittels Düsen ein wässriges Quenchmedium dem Spaltgas zugeführt und dieses schnell auf etwa 80 -90 °C abgekühlt. Der Prozess wird durch geeignete Wahl der Sauerstoffzahl λ so betrieben (λ < 0,31), wobei man unter der Sauerstoffzahl λ das Verhältnis aus der tatsächlich im zweiten Einsatzstrom vorhandenen Sauerstoffmenge zur stöchiometrisch notwendigen Sauerstoffmenge versteht, dass die Ausbeute an Acetylen bezogen auf das trockene Spaltgas optimal groß wird (> 8 %). Hierbei wird unter Sauerstoffzahl λ wie üblich das Verhältnis aus der tatsächlich vorhandenen Sauerstoffmenge zur stöchiometrisch notwendigen Sauerstoffmenge verstanden, die für die vollständige Verbrennung der Einsatzstoffe erforderlich ist. Hierbei wird jedoch auch die Rußbeladung des Spaltgases maximal. Der im Feuerraum aus der Gasphase gebildete Ruß wird zum Teil durch den Quench, in einer anschließenden Kühlkolonne und einem daran anschließenden Elektrofilter abgeschieden. Der wertprodukthaltige Produktgasstrom wird getrennt über die Kühlkolonne abgeführt. Nach dem Elektrofilter ist die Rußkonzentration im restlichen Spaltgas (ohne Wertprodukte) auf etwa 1 mg/m³ gesunken. Der im Prozesswasser aus dem Quench, der Kühlkolonne und dem Elektrofilter enthaltene Ruß besitzt einen hohen Kohlenwasserstoffanteil und ist daher hydrophob, was ihn auf dem Prozesswasser aufschwimmen lässt. Daher wird dieses rußbeladene Prozesswasser über sogenannte offene Rußrinnen mit Oberflächen-Partikelabscheidern geleitet. Die aufschwimmenden Rußanteile werden dabei abgetrennt und einer Feuerung zugeführt. Das so gereinigte Prozesswasser wird anschließend über einen offenen Kühlturm gefahren und somit abgekühlt. Dabei und während der Fest-Flüssigtrennung zuvor wird ein Großteil der flüssig und gasförmig im Prozesswasser gebundenen Kohlenwasserstoffe, insbesondere Aromate, Alkine, Benzol-Toluol-Xylol, etc. zusammen mit Teilen des Prozesswassers in die Umgebungsluft emittiert. Anschließend wird der so entstandene Verlust an Prozesswasser durch Zugabe kompensiert und der Wasserkreislauf Richtung Kühlkolonne und Quench geschlossen. Die Emissionen an Kohlenwasserstoffen aus dem Prozesswasser aus dem Kühlturm (d.h. bei einer offenen Prozesswasserfahrweise) sind jedoch unter den geltenden Umweltschutzauflagen nicht mehr tragbar. Bei einer geschlossenen Prozesswasserfahrweise würden sich aber die Kohlenwasserstoffe anreichern und zu Polymerisation und Verstopfung der Anlage führen, so dass auch eine geschlossene Prozesswasserfahrweise keine tragbare Lösung ist. Eine weitere Emissionsquelle stellen die offenen Rußrinnen dar.

Ein weiteres Verfahren zur Herstellung von Acetylen und Synthesegas durch partielle Oxidation von Kohlenwasserstoffen mit Sauerstoff ist in US 005824834A beschrieben. Dabei handelt es sich um ein Rußmengen-optimiertes, geschlossenes Wasserquenchverfahren, das mit einem mageren Feedstrom betrieben wird, und zwar mit einem Feedstrom mit einer Sauerstoffzahl λ < 0,31. Das Verfahren hat jedoch den Nachteil einer reduzierten Ausbeute an Wertprodukt Acetylen.

Bei dieser Verfahrensvariante wird das wässrige Quenchmedium ebenfalls mittels Düsen das dem Spaltgas zugeführt und dieses schnell auf etwa 80 -90 °C abgekühlt. Der im Feuerraum aus der Gasphase gebildete Ruß wird zum Teil durch den Quench, eine anschließende mit rezirkulierendem Wasser betriebene Kühlkolonne und einem daran anschließenden Elektrofilter abgeschieden. Der werthaltige Produktgasstrom wird getrennt über die Kühlkolonne abgeführt. Der Prozess wird hierbei durch Wahl der Sauerstoffzahl λ so betrieben (λ > 0,31), dass die anfallende Rußmenge im Spaltgas so gering ist, dass alleine durch die Ausschleusung des anfallenden Reaktionswassers aus der Verbrennung der stationäre Betrieb gewährleistet werden kann. Dadurch reduziert sich jedoch der Acetylengehalt im trockenen Spaltgas um 2 Prozentpunkte gegenüber dem vorstehend beschriebenen Verfahren auf etwa 6 Vol.-%. Damit wird eine geschlossene, also gegenüber der Umwelt abgetrennte Wasserquench-Fahrweise ermöglicht. Der Vorteil gegenüber der zuvor beschriebenen Verfahrensvariante ist somit die Möglichkeit des geschlossenen Betriebes ohne weitere Trennapparaturen. Der Nachteil sind Ausbeuteeinbußen bezüglich des Wert- und Zielprodukts Acetylen.

Es war demgegenüber Aufgabe der Erfindung, ein Verfahren zur Herstellung von Acetylen und Synthesegas durch partielle Oxidation von Kohlenwasserstoffen zur Verfügung zu stellen, das die Vorteile der beiden obigen Verfahren kombiniert, das heißt sowohl eine hohe Ausbeute an Wertprodukt Acetylen als auch die Einhaltung der geltenden Umweltschutzauflagen gewährleistet.

Die Aufgabe wird gelöst durch ein Verfahren zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter, Sauerstoff enthaltender Einsatzstrom
- getrennt voneinander vorgeheizt,
   in einem Verhältnis der Massenströme des zweiten Einsatzstromes zum ersten Einsatzstrom entsprechend einer Sauerstoffzahl λ von kleiner oder gleich 0,31 gemischt werden, die für die vollständige Verbrennung des einen oder der mehreren, im ersten Einsatzstrom enthaltenen Kohlenwasserstoffe, erforderlich ist,
- über einen Brennerblock einem Feuerraum zugeführt werden, worin die partielle Oxidation der Kohlenwasserstoffe stattfindet,
- unter Erhalt eines Spaltgases, das stromabwärts des Feuerraumes durch Eindüsen eines wässrigen Quenchmediums auf 80 bis 90 °C gequencht wird, wobei
   - ein Prozesswassersstrom I_{liq}, sowie
   - ein Produktgasstrom I_{g} erhalten wird, der
   - in einer Kühlkolonne durch direkten Wärmetausch mit Kühlwasser abgekühlt wird, unter Erhalt
   - eines Prozesswasserstromes II_{liq} als Sumpfstrom,
   - eines Produktgasstromes II_{g} als Kopfstrom, sowie
   - eines Seitenstromes, der
- in einem Elektrofilter an Ruß abgereichert wird und wobei im Elektrofilter ein Prozesswasserstrom III_{liq} anfällt, wobei
- die Prozesswasserströme I_{liq}, II_{liq} und III_{liq} vereinigt und über Rußrinnen mit Oberflächen-Partikelabscheidern geleitet werden, unter Erhalt eines vereinigten Prozesswasserstromes IV_{liq},
   dadurch gekennzeichnet, dass
der vereinigte Prozesswasserstrom IV_{liq} einer Reinigung durch Teilverdampfung in einem einstufigen Entspannungsbehälter unterworfen wird, wobei der vereinigte Prozesswasserstrom IV_{liq} zu einem Anteil von 0,01 Gew.-% bis 10 Gew.-% bezogen auf das Gesamtgewicht desselben, verdampft wird, unter Erhalt eines gereinigten Prozesswasserstromes V_{liq}, der in das Verfahren recycliert wird.

Es wurde gefunden, dass durch eine Teilverdampfung der vereinigten Prozesswasserströme in einem einstufigen Entspannungsbehälter die unerwünschten gelösten Gase, insbesondere polymerisierbare Komponenten, beispielsweise höhere Acetylene, aus den Prozesswasserströmen mit dem Entspannungsdampf in die Gasphase mitgerissen werden, und soweit von der Flüssigphase, dem vereinigten Prozesswasserstrom, abgetrennt werden können, dass dieser in das Verfahren recycliert von welchem auch das überschüssige, anfallende Abwasser entsorgt werden kann.

Die mit dem Entspannungsdampf mitgerissenen Dämpfe von unerwünschten gelösten Gasen können anschließend zum Beispiel nach Kondensation des Wasserdampfes verbrannt oder anderweitig im Prozess entsorgt werden.

Es hat sich überraschend gezeigt, dass eine einstufige Entspannung zur Teilverdampfung des vereinigten Prozesswasserstromes, zu einem Anteil von 0,01 bis 10 Gew.-%, bezogen auf das Gesamtgewicht des vereinigten Prozesswasserstromes eine ausreichende Abreicherung an unerwünschten gelösten Komponenten ermöglicht, so dass das Verfahren in einem geschlossenen Prozesswasserkreislauf betrieben werden kann.

In einer bevorzugten Ausführungsform wird das Verfahren mit einem geschlossenen Prozesswasserkreislauf betrieben. In dieser Verfahrensvariante wird bevorzugt ein Teilstrom des gereinigten Prozesswasserstromes aus dem Verfahren ausgeschleust und der übrige Teilstrom des gereinigten Prozesswasserstromes in das Verfahren recycliert.

In einer weiteren bevorzugten Verfahrensvariante wird der gereinigte Prozesswasserstrom in einem offenen Kühlturm abgekühlt. In dieser Verfahrensvariante wird bevorzugt der gesamte gereinigte Prozesswasserstrom in das Verfahren recycliert.

Bevorzugt wird der vereinigte Prozesswasserstrom zu einem Anteil von 1 Gew.-% bis 2 Gew.-%, bezogen auf das Gesamtgewicht desselben, verdampft.

Das Verfahren zur Herstellung von Acetylen und Synthesegas wird erfindungsgemäß mit einer Sauerstoffzahl λ von kleiner oder gleich 0,31 betrieben, wobei unter Sauerstoffzahl λ das Verhältnis aus der tatsächlich im zweiten Einsatzstrom vorhandenen Sauerstoffmenge zur stöchiometrisch notwendigen Sauerstoffmenge verstanden wird, die für die vollständige Verbrennung des einen oder der mehreren, im ersten Einsatzstrom enthaltenen Kohlenwasserstoffe erforderlich ist.

Bei einem Betrieb mit einer Sauerstoffzahl λ im obigen Bereich wird eine hohe Ausbeute an Wertprodukt Acetylen gewährleistet.

Das Verfahren ist unabhängig von der konkreten Ausbildung des Reaktorsystems umfassend Mischeinrichtung, Brennerblock sowie die Quencheinrichtung.

Im Folgenden werden die üblicherweise eingesetzten Reaktorsysteme näher erläutert: Die Ausgangsstoffe, das heißt ein Kohlenwasserstoffe enthaltender Gasstrom, insbesondere Erdgas, und Sauerstoff, werden dabei getrennt aufgeheizt, üblicherweise bis hin zu 600 °C. In einer Mischeinrichtung werden die Reaktanden intensiv vermischt und nach Durchströmen eines Brennerblocks zur exothermen Reaktion gebracht. Der Brennerblock besteht üblicherweise aus einer Vielzahl von parallelen Kanälen, in denen die Strömungsgeschwindigkeit der zündfähigen Sauerstoff/KohlenwasserstoffMischung höher ist als die Flammengeschwindigkeit, um ein Durchschlagen der Flamme in die Mischeinrichtung zu verhindern. Der metallische Brennerblock wird gekühlt, um den thermischen Belastungen standzuhalten. Je nach Aufenthaltszeit in der Mischeinrichtung besteht die Gefahr der Vor- und Rückzündung aufgrund der begrenzten thermischen Stabilität der Mischungen. Hierzu wird der Begriff der Zündverzugszeit, bzw. Induktionszeit gebraucht als die Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden in der Mischeinrichtung. Reaktanden wie Wasserstoff, Flüssiggas oder Leichtbenzin, deren Einsatz aufgrund von Ausbeute- und /oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch die zylinderförmige Geometrie des Feuerraums aus. Der Brennerblock weist vorzugsweise hexagonal angeordnete Durchführungsbohrungen auf. In einer Ausführungsform sind z.B. 127 Bohrungen ä 27 mm Innendurchmesser hexagonal auf einem kreisförmigen Grundquerschnitt mit Durchmesser von ca. 500 mm angeordnet. In der Regel liegen die eingesetzten Kanaldurchmesser bei etwa 19 bis 27 mm Durchmesser. Der anschließende Feuerraum, in dem die Flamme der acetylenbildenden partiellen Oxidationsreaktion stabilisiert wird, ist üblicherweise ebenfalls von zylindrischem Querschnitt, ist wassergekühlt und entspricht im Erscheinungsbild dem eines kurzen Rohres (von z.B. 180 bis 533 mm Durchmesser und 380 bis 450 mm Länge). In Höhe des Brennerblocks wird sowohl in axialer als auch in radialer Richtung sogenannter Hilfssauerstoff dem Feuerraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt. Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind am äußeren Umfang derselben Quenchdüsen auf einem oder mehreren Quenchverteilerringen installiert, die das Quenchmedium mit oder ohne Zuhilfenahme eines Zerstäubungsmedium zerstäuben und näherungsweise senkrecht zur Hauptströmungsrichtung der den Feuerraum verlassenden Reaktionsgase eindüsen. Dieser direkte Quench hat die Aufgabe das Reaktionsgemisch extrem schnell abzukühlen, so dass Folgereaktionen, d.h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Bei dem vorliegenden technischen Verfahren entsteht neben Acetylen im Wesentlichen Wasserstoff, Kohlenmonoxid und Ruß. Die in der Flammenfront gebildeten Rußpartikel können als Keime an den Feuerraum-Seitenwänden anhaften, worauf es bei geeigneten physikalisch-chemischen Bedingungen zum Aufwachsen, Ablagern und Anbacken von Koksschichten kommt.
Diese Ablagerungen werden periodisch im Bereich der Feuerraumwände mittels einer Stocherreinrichtung mechanisch abgereinigt.

Die vorliegende Erfindung nutzt den Umstand, dass beim obigen Wasserquenchverfahren ein Prozesswasserstrom I_{liq} (ein Quenchwasser) bei einer Temperatur im Bereich zwischen 60 und 96°C, bevorzugt mit einer Temperatur im Bereich von circa 70 bis 80°C, anfällt. Die enthaltene thermische Energie erlaubt eine ausreichende Abtrennung von unerwünschten gelösten Gasen durch Teilverdampfung ins Vakuum.

Bevorzugt erfolgt die Teilverdampfung durch einstufige Entspannung ins Vakuum.

Weiter bevorzugt erfolgt die Teilverdampfung durch einstufige Entspannung adiabat.

In einer Verfahrensvariante kann die Teilverdampfung vorteilhaft durch Wärmeeintrag unterstützt werden.

In einer weiteren bevorzugten Verfahrensvariante sind die Rußrinnen mit Oberflächen-Partikelabscheidern, über die die vereinigten Prozesswasserströme I, II und III geleitet werden, mit einer Abdeckung versehen.

Eine hinreichende Abtrennung der gelösten Gase kann auch durch eine Strippkolonne erreicht werden. Hierzu wird der vereinigte Prozesswasserstrom auf den Kopf der Kolonne und der Strippdampf im Gegenstrom in den Sumpf der Strippkolonne gegeben. Auch mit diesem Verfahrensschritt wird eine ausreichende Abreicherung der gelösten Gase erreicht. Der apparative Aufwand und somit auch die Investitionskosten des verfahrenstechnischen Schritts sind deutlich höher als beim einfachen, erfindungsgemäßen Flash. Außerdem neigen die Einbauten der dann notwendigen Trennstufen und Verteiler deutlich mehr zum Verschmutzen durch polymerisierende Komponenten als der einfache Aufbau einer einstufigen Entspannung.

Der Entspannungsbehälter ist bevorzugt einstufig und kann mit üblichen Einbauten, wie Packungen oder Böden, wie auch mit einem Demister gegen Tröpfchenmitriss ausgestattet sein.

Möglich ist auch eine mehrstufige Entspannung oder ein Wärmeeintrag im Sumpf wie bei einer Destillationskolonne statt Vorheizen des Zulaufs.

Somit stellt dieses Verfahren eine sehr kostengünstige Möglichkeit der Kreislaufwasserreinigung, bzw. Abwasserreinigung dar.

Das Vakuum kann nach im Stand der Technik bekannter Weise z. B. über eine Dampfstrahlanlage oder einen Wasserringverdichter erzeugt werden. Das Abgas lässt sich dann innerhalb der Anlage weiter behandeln oder auch einer Abgasverbrennung zuführen.

Die Erfindung wird im Folgenden anhand einer Zeichnung sowie in Ausführungsbeispielen näher erläutert.

In der Zeichnung zeigen im Einzelnen:
Figur 1 die schematische Darstellung einer bevorzugten erfindungsgemäßen Anlage mit Kühlturm und
Figur 2 die schematische Darstellung einer weiteren bevorzugten Anlage zur Durchführung des erfindungsgemäßen Verfahrens ohne Kühlturm.

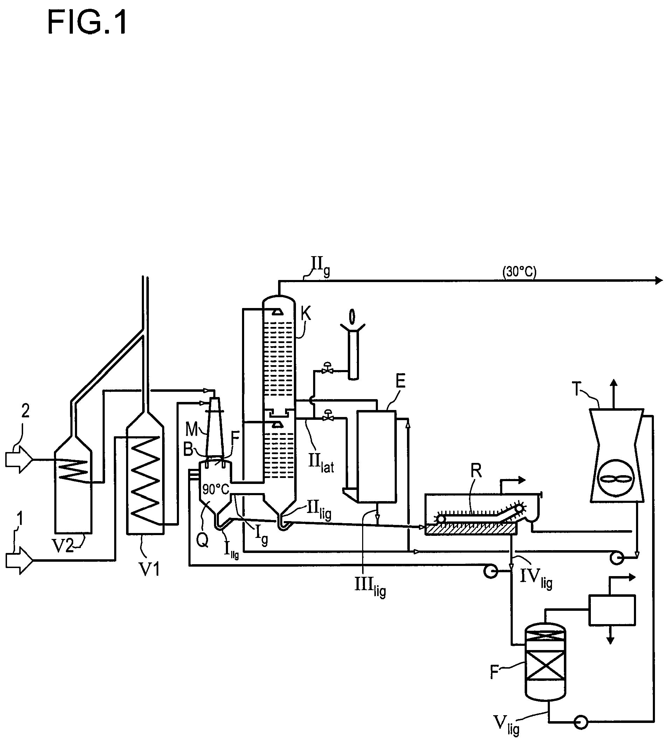

Der in Figur 1 dargestellten Anlage wird ein Kohlenwasserstoffe enthaltender Gasstrom (1) sowie ein Sauerstoff enthaltender Gasstrom (2) zugeführt, über Vorheizer V1 bzw. V2, voneinander getrennt vorgeheizt, in einer Mischeinrichtung (M) gemischt, über einen Brennerblock (B) einem Feuerraum (F) zugeführt und anschließend in einem Quenchbereich (Q) durch Eindüsen eines wässrigen Quenchmediums gequencht, wobei ein Prozesswasserstrom I_{liq} sowie ein Produktgasstrom I_{g} erhalten werden.

Der Produktgasstrom I_{g} wird in einer Kühlkolonne (K) durch direkten Wärmetausch mit Kühlwasser abgekühlt, unter Erhalt eines Prozesswasserstromes II_{liq} als Sumpfstrom, eines Produktgasstromes II_{g} als Kopfstrom sowie eines Seitenstromes IIₗₐₜ. Der Seitenstrom IIₗₐₜ wird einem Elektrofilter (E) zugeführt und dort an Ruß abgereichert, wobei ein Prozesswasserstrom III_{liq} entsteht. Die Prozesswasserströme I_{liq}, II_{liq} und III_{liq} werden vereinigt und über Rußrinnen (R) mit Oberflächen-Partikelabscheidern geleitet, unter Erhalt eines vereinigten Prozesswasserstromes IV_{liq}. Dieser wird einem einstufigen Entspannungsbehälter (F) zugeführt und darin teilverdampft, unter Erhalt eines gereinigten Prozesswasserstromes V_{liq}, der in einem Kühlturm (T) abgekühlt und in das Verfahren, in die Kühlkolonne (K), recycliert wird.

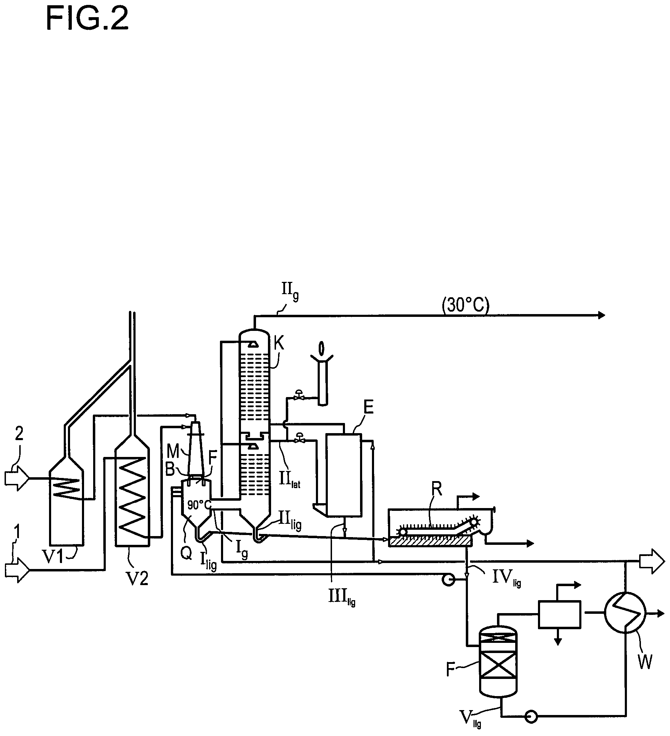

Die in Figur 2 dargestellte weitere bevorzugte Ausführungsform zeigt eine weitgehend analoge Anlage, wobei jedoch anstelle des Kühlturmes (T) ein Wärmetauscher (W) vorgesehen ist, über den der vereinigte Prozesswasserstrom V_{liq} gekühlt und erneut in das Verfahren, in die Kühlkolonne (K), recycliert wird.

### Ausführungsbeispiele:

### Vergleichsbeispiel:

Ohne Prozesswasserreinigung ergeben sich in einer Anlage entsprechend der schematischen Darstellung in Figur 1 spezifisch für 1 t Acetylen folgende Emissionen aus den offenen Rußrinnen und der Abluft des Kühlturms:

| **Emissionen offener Wasserquench** | | | |
|---|---|---|---|
| | Rußrinnen [kg] | Kühlturm [kg] | gesamt [kg] |
| CO | 0,303 | 0,363 | 0,667 |
| Methan | 5,67E-02 | 8,46E-02 | 0,141 |
| Ethan | 7,63E-03 | 1,21E-02 | 0,020 |
| Ethylen | 6,80E-03 | 2,88E-02 | 0,036 |
| Acetylen | 1,57E-01 | 6,05E+00 | 6,203 |
| Propen | 5,16E-04 | 1,88E-03 | 0,002 |
| Propadien | 9,83E-04 | 3,58E-03 | 0,005 |
| Propin | 2,29E-03 | 1,01E-01 | 0,103 |
| Butenin | 1,65E-03 | 4,58E-02 | 0,047 |
| Butadiin | 7,39E-03 | 8,91E-01 | 0,898 |
| Benzol | 2,29E-03 | 1,60E-01 | 0,162 |
| Naphthalin | 5,14E-04 | 1,40E-02 | 0,014 |

### Ausführungsbeispiele nach der Erfindung:

Die Abreinigungseffizienz des Prozesswassers ist eine Funktion der Flashdampfmenge wie in der folgenden Tabelle dargestellt:
Dazu wird das Prozesswasser ausgehend von 84,4°C und 1.013 bar absolut auf Drücke zwischen 200 mbar absolut und 800 mbar absolut entspannt. Dabei wird das Prozesswasser zu einem Anteil von 0,0013 Gew. % bis 4,18 Gew. % teilverdampft. Es ergeben sich als Funktion des Entspannungsdrucks folgende Abreicherungen an gelösten Gasen.

| **Abreicherung durch Flash in Abhängigkeit des Drucks (offener Wasserquench)** | | | | |
|---|---|---|---|---|
| Temperatur Austritt [°C] | 84,409 | 84,2 | 75,8 | 60 |
| Temperatur Eintritt [°C] | 84,41 | 84,4 | 84,4 | 84,4 |
| Druck Eintritt [bar absolut] | 1,013 | 1,013 | 1,013 | 1,013 |
| Druck Austritt [mbar absolut] | 800 | 600 | 400 | 200 |
| Flashdampfmenge bezogen auf Zulauf [%] | 0,0013 % | 0,0337 % | 1,44 % | 4,18 % |
| | Abreicherung | Abreicherung | Abreicherung | Abreicherung |
| CO | 78,1 % | 98,9 % | 99,98 % | 100,00 % |
| Methan | 72,7 % | 98,5 % | 99,97 % | 100,00 % |
| Ethan | 71,2 % | 98,3 % | 99,97 % | 99,99 % |
| Ethylen | 43,1 % | 94,4 % | 99,90 % | 99,98 % |
| Acetylen | 7,1 % | 60,6 % | 98,93 % | 99,79 % |
| Propen | 47,4 % | 95,3 % | 99,92 % | 99,98 % |
| Propadien | 47,4 % | 95,3 % | 99,92 % | 99,98 % |
| Propin | 6,4 % | 57,3 % | 98,67 % | 99,71 % |
| Butenin | 9,6 % | 68,3 % | 99,18 % | 99,83 % |
| Butadien | 2,9 % | 32,9 % | 95,97 % | 98,91 % |
| Benzol | 4,2 % | 45,8 % | 97,93 % | 99,52 % |
| Naphthalin | 10,5 % | 71,9 % | 99,30 % | 99,85 % |

Es zeigt sich deutlich, dass die Abreicherung stark vom Entspannungsdruck abhängt.

Erfolgt eine erfindungsgemäße, z.B. einstufige Entspannung des Prozesswassers vor dem Kühlturm, so ergeben sich nur mehr folgende Emissionen an die Umwelt:
Das Prozesswasser tritt mit 84.4°C in die einstufige Flash-Stufe ein und wird dabei auf 400 mbar absolut entspannt.

Dabei kühlt sich der Strom von 84.4°C auf 75.8°C ab und es entstehen dabei 1.44 % Flash-Dampf bezogen auf den Zulauf.

In der Tabelle ist außerdem noch die Abreicherung durch den Reinigungsschritt in Prozenten angegeben.

| **Emissionen offener Wasserquench mit Flash** | | |
|---|---|---|
| | Kühlturm | Abreicherung |
| | Fluss kg/t Ac | in % |
| CO | 1,20E-04 | 99,9820 % |
| Methan | 3,53E-05 | 99,9750 % |
| Ethan | 5,39E-06 | 99,9726 % |
| Ethylen | 3,55E-05 | 99,9002 % |
| Acetylen | 6,67E-02 | 98,9253 % |
| Propen | 1,99E-06 | 99,9172 % |
| Propadien | 3,78E-06 | 99,9172 % |
| Propin | 1,37E-03 | 98,6727 % |
| Butenin | 3,90E-04 | 99,1785 % |
| Butadiin | 3,62E-02 | 95,9707 % |
| Benzol | 3,36E-03 | 97,9296 % |
| Naphthalin | 1,01E-04 | 99,3007 % |

Aufgrund der hohen Abreicherungsrate kann der Kühlturm durch einen geschlossenen Wärmetauscher ersetzt werden, ohne dass es im Prozess zu nicht tolerablen Aufpegelungen von polymerisierbaren Komponenten, insbesondere von höheren Acetylenen, kommt.

| | **Nebenkomponenten im Prozesswasser** | |
|---|---|---|
| | geschlossener Wasserquench ohne flash [Gew.ppm] | geschlossener Wasserquench mit flash [Gew.ppm] |
| CO | 1,846 | 0,001 |
| Methan | 0,430 | 0,000 |
| Ethan | 0,061 | 0,000 |
| Ethylen | 0,146 | 0,000 |
| Acetylen | 30,537 | 0,333 |
| Propen | 0,010 | 0,000 |
| Propadien | 0,018 | 0,000 |
| Propin | 0,514 | 0,007 |
| Butenin | 0,233 | 0,002 |
| Butadiin | 4,606 | 0,182 |
| Benzol | 0,018 | 0,017 |
| Naphthalin | 0,071 | 0,001 |

## Patentansprüche

1. Verfahren zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter, Sauerstoff enthaltender Einsatzstrom
- getrennt voneinander vorgeheizt,
in einem Verhältnis der Massenströme des zweiten Einsatzstromes zum ersten Einsatzstrom entsprechend einer Sauerstoffzahl λ von kleiner oder gleich 0,31 gemischt werden, wobei man unter der Sauerstoffzahl λ das Verhältnis aus der tatsächlich im zweiten Einsatzstrom vorhandenen Sauerstoffmenge zur stöchiometrisch notwendigen Sauerstoffmenge versteht, die für die vollständige Verbrennung des einen oder der mehreren, im ersten Einsatzstrom enthaltenen Kohlenwasserstoffe, erforderlich ist,
- über einen Brennerblock einem Feuerraum zugeführt werden, worin die partielle Oxidation der Kohlenwasserstoffe stattfindet,
- unter Erhalt eines Spaltgases, das stromabwärts des Feuerraumes durch Eindüsen eines wässrigen Quenchmediums auf 80 bis 90 Grad Celsius gequencht wird, wobei
- ein Prozesswassersstrom I_{liq}, sowie
- ein Produktgasstrom I_{g} erhalten wird, der
- in einer Kühlkolonne durch direkten Wärmetausch mit Kühlwasser abgekühlt wird, unter Erhalt
- eines Prozesswasserstromes II_{liq} als Sumpfstrom,
- eines Produktgasstromes II_{g} als Kopfstrom, sowie
- eines Seitenstromes, der
- in einem Elektrofilter an Ruß abgereichert wird und wobei im Elektrofilter ein Prozesswasserstrom III_{liq} anfällt, wobei
- die Prozesswasserströme I_{liq}, II_{liq} und III_{liq} vereinigt und über Rußrinnen mit Oberflächen -Partikelabscheidern geleitet werden, unter Erhalt eines vereinigten Prozesswasserstromes IV_{liq},
**dadurch gekennzeichnet, dass** der vereinigte Prozesswasserstrom IV_{liq} einer Reinigung durch Teilverdampfung in einem einstufigen Entspannungsbehälter unterworfen wird, wobei der vereinigte Prozesswasserstrom IV_{liq} zu einem Anteil von 0,01 Gew.-% bis 10 Gew.-%, bezogen auf das Gesamtgewicht desselben verdampft wird, unter Erhalt eines
gereinigten Prozesswasserstromes V_{liq},
der in das Verfahren recycliert wird.

2. Verfahren nach Anspruch 1, **dadurch gekennzeichnet, dass** der gereinigte Prozesswasserstrom V_{liq} vollständig in das Verfahren recycliert wird.

3. Verfahren nach Anspruch 1, **dadurch gekennzeichnet, dass** ein Teilstrom des gereinigten Prozesswasserstromes V_{liq} in das Abwasser entsorgt wird, und der übrige Teilstrom des gereinigten Prozesswasserstromes V_{liq} in das Verfahren recycliert wird.

4. Verfahren nach einem der Ansprüche 1 bis 3, **dadurch gekennzeichnet, dass** der vereinigte Prozesswasserstrom IV_{liq} zu einem Anteil von 1 Gew.-% bis 2 Gew.-%, bezogen auf das Gesamtgewicht desselben, verdampft wird.

5. Verfahren nach einem der Ansprüche 1 bis 4, **dadurch gekennzeichnet, dass** die Teilverdampfung durch Entspannung ins Vakuum erfolgt.

6. Verfahren nach Anspruch 4, **dadurch gekennzeichnet, dass** die Teilverdampfung durch Entspannung in ein Vakuum von 50 bis 900 mbar a erfolgt.

7. Verfahren nach Anspruch 4, **dadurch gekennzeichnet, dass** die Teilverdampfung durch Entspannung in ein Vakuum von 200 bis 600 mbar a erfolgt.

8. Verfahren nach einem der Ansprüche 1 bis 7, **dadurch gekennzeichnet, dass** die Teilverdampfung durch Entspannung adiabat erfolgt.

9. Verfahren nach einem der Ansprüche 1 bis 8, **dadurch gekennzeichnet, dass** die Teilverdampfung durch Wärmeeintrag unterstützt wird.

10. Verfahren nach Anspruch 9, **dadurch gekennzeichnet, dass** der Wärmeeintrag durch direkte Dampfeinspeisung erfolgt.

11. Verfahren nach einem der Ansprüche 1 bis 10, **dadurch gekennzeichnet, dass** die Rußrinnen mit Oberflächen-Partikelabscheidern mit einer Abdeckung versehen sind.

## Claims

1. A process for preparing acetylene and synthesis gas by partial oxidation of hydrocarbons with oxygen, in which a first input stream comprising one or more hydrocarbons and a second input stream comprising oxygen
- are separately preheated,
mixed in a ratio of the mass flow rates of the second input stream to the first input stream corresponding to an oxygen ratio λ of less than or equal to 0.31, the oxygen ratio λ being understood to mean the ratio of the amount of oxygen actually present in the second input stream to the stochiometrically necessary amount of oxygen required for the complete combustion of the one or more hydrocarbons present in the first input stream,
- supplied via a burner block to a combustion chamber in which the partial oxidation of the hydrocarbons takes place
- to obtain a cracking gas which is quenched to 80 to 90 degrees Celsius downstream of the combustion chamber by injection of an aqueous quench medium, to obtain
- a process water stream I_{liq} and
- a product gas stream I_{g} which
- is cooled in a cooling column by direct heat exchange with cooling water to obtain
- a process water stream II_{lig} as the bottom stream,
- a product gas stream II_{g} as the top stream and
- a side stream which
- is depleted of soot in an electrostatic filter to obtain a process water stream III_{liq} in the electrostatic filter, and
- process water streams I_{liq}, II_{liq} and III_{liq} are combined and passed through soot channels with surface particulate precipitators to obtain a combined process water stream IV_{liq},
which comprises subjecting the combined process water stream IV_{liq} to a cleaning operation by partial vaporization in a one-stage flash vessel, the combined process water stream IV_{liq} being vaporized in a proportion of 0.01% by weight to 10% by weight, based on the total weight thereof, to obtain a
cleaned process water stream V_{liq},
which is recycled into the process.

2. The process according to claim 1, wherein the cleaned process water stream V_{liq} is fully recycled into the process.

3. The process according to claim 1, wherein a substream of the cleaned process water stream V_{liq} is disposed of as wastewater, and the remaining substream of the cleaned process water stream V_{liq} is recycled into the process.

4. The process according to any of claims 1 to 3, wherein the combined process water stream IV_{liq} is evaporated in a proportion of 1% by weight to 2% by weight, based on the total weight thereof.

5. The process according to any of claims 1 to 4, wherein the partial vaporization is effected by flashing into vacuum.

6. The process according to claim 4, wherein the partial vaporization is effected by flashing into a vacuum of 50 to 900 mbar a.

7. The process according to claim 4, wherein the partial vaporization is effected by flashing into a vacuum of 200 to 600 mbar a.

8. The process according to any of claims 1 to 7, wherein the partial vaporization by flashing is effected adiabatically.

9. The process according to any of claims 1 to 8, wherein the partial vaporization is promoted by heat input.

10. The process according to claim 9, wherein the heat input is effected by direct steam injection.

11. The process according to any of claims 1 to 10, wherein the soot channels with surface particulate precipitators are provided with a cover.

## Revendications

1. Procédé de fabrication d'acétylène et d'un gaz de synthèse par oxydation partielle d'hydrocarbures avec de l'oxygène, selon lequel un premier courant d'entrée, contenant un ou plusieurs hydrocarbures, et un second courant d'entrée, contenant de l'oxygène,
- sont préchauffés séparément l'un de l'autre,
et mélangés en un rapport entre les débits massiques du second courant d'entrée et du premier courant d'entrée correspondant à un indice d'oxygène λ inférieur ou égal à 0,31, l'indice d'oxygène λ se rapportant au rapport entre la quantité réelle d'oxygène présente dans le second courant d'entrée et la quantité d'oxygène stoechiométriquement nécessaire pour la combustion totale du ou des hydrocarbures contenus dans le premier courant d'entrée,
- sont introduits dans une enceinte chauffante au-dessus d'un bloc brûleur, où l'oxydation partielle des hydrocarbures a lieu,
- pour obtenir un gaz de clivage, qui est trempé à 80 à 90 degrés Celsius en aval de l'enceinte chauffante par injection d'un milieu de trempe aqueux, pour obtenir
- un courant d'eau de procédé I_{liq} et
- un courant gazeux de produits I_{g}, qui
- est refroidi dans une colonne de refroidissement par échange de chaleur direct avec une eau de refroidissement, pour obtenir
- un courant d'eau de procédé II_{liq} en tant que courant de fond,
- un courant gazeux de produits II_{g} en tant que courant de tête, et
- un courant latéral, qui
- est appauvri en noir de carbone dans un électrofiltre, un courant d'eau de procédé III_{liq} se formant dans l'électrofiltre,
- les courants d'eau de procédé I_{liq}, II_{liq} et III_{liq} étant réunis et conduits par des rainures de noir de carbone munies de séparateurs de particules de surface, pour obtenir un courant d'eau de procédé réuni IV_{liq},
**caractérisé en ce que**
le courant d'eau de procédé réuni IV_{liq} est soumis à une purification par évaporation partielle dans un contenant de détente à une étape, le courant d'eau de procédé réuni IV_{liq} étant évaporé jusqu'à une proportion de 0,01 % en poids à 10 % en poids, par rapport à son poids total, pour obtenir un courant d'eau de procédé purifié V_{liq}, qui est recyclé dans le procédé.

2. Procédé selon la revendication 1, **caractérisé en ce que** le courant d'eau de procédé purifié V_{liq} est recyclé en totalité dans le procédé.

3. Procédé selon la revendication 1, **caractérisé en ce qu'**un courant partiel du courant d'eau de procédé purifié V_{liq} est mis au rebut dans l'eau résiduaire, et l'autre courant partiel du courant d'eau de procédé purifié V_{liq} est recyclé dans le procédé.

4. Procédé selon l'une quelconque des revendications 1 à 3, **caractérisé en ce que** le courant d'eau de procédé réuni IV_{liq} est évaporé jusqu'à une proportion de 1 % en poids à 2 % en poids, par rapport à son poids total.

5. Procédé selon l'une quelconque des revendications 1 à 4, **caractérisé en ce que** l'évaporation partielle par détente a lieu sous vide.

6. Procédé selon la revendication 4, **caractérisé en ce que** l'évaporation partielle par détente a lieu dans un vide de 50 à 900 mbar a.

7. Procédé selon la revendication 4, **caractérisé en ce que** l'évaporation partielle par détente a lieu dans un vide de 200 à 600 mbar a.

8. Procédé selon l'une quelconque des revendications 1 à 7, **caractérisé en ce que** l'évaporation partielle par détente a lieu de manière adiabatique.

9. Procédé selon l'une quelconque des revendications 1 à 8, **caractérisé en ce que** l'évaporation partielle est soutenue par un apport de chaleur.

10. Procédé selon la revendication 9, **caractérisé en ce que** l'apport de chaleur a lieu par introduction directe de vapeur.

11. Procédé selon l'une quelconque des revendications 1 à 10, **caractérisé en ce que** les rainures de noir de carbone munies de séparateurs de particules de surface sont équipées d'un couvercle.
